# EUROPEAN PATENT SPECIFICATION

(11) **EP 4 252 741 B1**
(45) Date of publication and mention of the grant of the patent: **06.08.2025**
(21) Application number: 22908821.6
(22) Date of filing: 18.11.2022
(51) Int. Cl.: A61K 9/16, A61K 38/09, A61M 37/00

(54) **MICRONEEDLE COMPRISING HORMONE-CONTAINING SURFACE-MODIFIED MICROSPHERES AND METHOD FOR MANUFACTURING SAME**
MIKRONADEL MIT HORMONHALTIGEN OBERFLÄCHENMODIFIZIERTEN MIKROKUGELN UND VERFAHREN ZUR HERSTELLUNG DAVON
MICRO-AIGUILLE COMPRENANT DES MICROSPHÈRES MODIFIÉES EN SURFACE CONTENANT DES HORMONES ET SON PROCÉDÉ DE FABRICATION

(30) Priority: 31.12.2021 KR 20210194329; 31.10.2022 KR 20220143147
(43) Date of publication of application: 04.10.2023
(73) Proprietor: Small'Lab Co., Ltd., Daejeon 34-027 (KR)
(72) Inventor: JOO, So Kyoung, Seongnam-si, Gyeonggi-do 13490 (KR); KIM, Byeong Su, Daejeon 34011 (KR); LEE, Jeong Gyu, Daejeon 34049 (KR); PARK, Chun Woong, Sejong 30063 (KR); HAN, Chang Soo, Cheongju-si, Chungcheongbuk-do 28160 (KR); CHOI, Jae Cheol, Chenogju-si, Chungcheongbukdo 28222 (KR)
(74) Representative: Ter Meer Steinmeister & Partner
(86) International application number: PCT/KR2022/018362
(87) International publication number: WO 2023/128281

(56) References cited:
- KR-A- 20080 050 580
- KR-B1- 101 549 086
- KR-B1- 101 549 086
- US-A1- 2014 343 499
- US-A1- 2021 178 138
- SHI N.-Q. ET AL.: "Microencapsulation of luteinizing hormone-releasing hormone agonist in poly (lactic-co-glycolic acid) microspheres by spray-drying", JOURNAL OF CONTROLLED RELEASE, ELSEVIER, AMSTERDAM, NL, vol. 321, 11 January 2020 (2020-01-11), pages 756 - 772, XP086135609, ISSN: 0168-3659, [retrieved on 20200111], DOI: 10.1016/J.JCONREL.2020.01.023
- SHI NIAN-QIU; ZHOU JIA; WALKER JENNIFER; LI LI; HONG JUSTIN K.Y.; OLSEN KARL F.; TANG JIE; ACKERMANN ROSE; WANG YAN; QIN BIN; SCHW: "Microencapsulation of luteinizing hormone-releasing hormone agonist in poly (lactic-co-glycolic acid) microspheres by spray-drying", JOURNAL OF CONTROLLED RELEASE, ELSEVIER, AMSTERDAM, NL, vol. 321, 11 January 2020 (2020-01-11), AMSTERDAM, NL , pages 756 - 772, XP086135609, ISSN: 0168-3659, DOI: 10.1016/j.jconrel.2020.01.023
- OGUNJIMI ABAYOMI T., FIEGEL JENNIFER, BROGDEN NICOLE K.: "Design and Characterization of Spray-Dried Chitosan-Naltrexone Microspheres for Microneedle-Assisted Transdermal Delivery", PHARMACEUTICS, vol. 12, no. 6, 29 May 2020 (2020-05-29), pages 496, XP093075320, DOI: 10.3390/pharmaceutics12060496

## Description

### Technical Field

The present invention relates to microneedles comprising hormone-containing surface-modified microspheres, and a method of preparing the same, and more particularly, to microneedles including surface-modified microspheres which exhibit improved residence time in the body and superior sustained-release effect, and are suitable for hormone delivery requiring continuous drug administration due, and a method of preparing the same.

### Background Art

Hormones generally refer to a group of chemical substances produced in the endocrine system in the body. Although not substantially different from neurotransmitters, substances that are secreted from a wide range of endocrine organs and act in a wide range for relatively a long time through blood, compared to neurotransmitters moving through the central nervous system as a main route, are called hormones. Hormones produced in various endocrine organs are delivered to various organs of the body through blood vessels, where they exerts their own functions. Particularly, it is known that hormones are directly involved in metabolism, reproduction, and cell proliferation. Therefore, hormones need to be secreted within a normal range in the body. In case of hormone deficiency, artificial supplementation is needed. The most common approach for hormone supplementary therapy is administration through injections. However, in case of conventional subcutaneous or muscular injections with short half-lives, rapid decrease in blood hormone concentration may occur after administration. As a result, there has been an inconvenience of daily administration required to maintain the therapeutic effect of hormones, and this inconvenience has been further exacerbated by the characteristic of injections.

The delivery of drugs through the skin, also known as transdermal drug delivery, is widely used in various fields and forms due to its convenience. Transdermal drug delivery is primarily used to deliver drugs through the skin into the systemic circulation. However, it is also utilized for delivering drugs, for example, drugs for atopic dermatitis, acne treatment, or skin disease treatment, within the skin itself. Despite such convenience and functionality, since there are many difficulties in delivering drugs through the skin due to its structure barriers, it is not easy to develop drugs passing through the skin. The cornified layer of the skin (stratum corneum) includes a brick structure consisting of keratin-rich keratinocytes and a mortar structure that fills the space between these keratinocytes with lipids such as ceramides, fatty acids, or wax. This structure acts as a barrier, resulting in very low permeability to substances. Only small substances with a low molecular weight of 500 Da or less may be delivered into the skin by diffusion, and only substances with excellent lipophilicity can permeate the skin.

To overcome the above problem, new systems such as microneedles are being developed, and microneedles can be applied in the form of a patch without any accessory equipment, making them convenient for daily use. Among the systems, a microneedle patch comprising multiple microneedles attached thereto, and these microneedles create small punctures in the skin surface to deliver drugs.

Recently, soluble microneedles based on a biodegradable polymer have been developed. After these microneedles penetrate into the skin and are biodegraded in the body, active ingredients are eluted into the skin (Korean Patent No. 10-2234446). However, the active ingredients permeating into the skin by the soluble microneedles may be escaped from subcutaneous tissue due to skin elasticity, resulting in reduced residence time in the body. Sustained-release drugs in which it is necessary for an active ingredient to exhibit its effectiveness for a long time particularly need higher residence time in the body.

Accordingly, there is a demand for the development of microneedles including hormone-containing surface-modified microspheres with improved residence time in the body and superior sustained-release effect. KR 101 549 086 B1 relates to a micro-needle for transdermal delivery of an active material having pharmaceutical, medical, or cosmetic effects, and to a micro-needle patch. The micro-needle includes a micro-needle-shaped biocompatibility matrix; and porous particles provided on the surface or at least one part of the inside of the biocompatibility matrix. US 2021/178138 A1 relates to an implantable sustained-release microneedle patch and a preparation method therefor. The implantable sustained-release microneedle comprises a needle tip, a needle body, and a base. The needle tip comprises a needle tip center layer and a needle tip outer layer. The needle tip center layer is formed of a matrix comprising a biodegradable water-insoluble polymer material. The needle tip outer layer, the needle body, and the base are formed of a matrix comprising a water-soluble polymer material. The microneedle patch comprises the microneedle and absorbs the moisture of the skin when the microneedle acts on skin, so that the base and the needle body are quickly separated from the needle tip within 0.5-2 h. After the base is removed, the needle tip will be left in skin, and the user does not need the patch sticking for a long time to ensure long-term release of a drug in the body. SHI N.-Q. et al. in "Microencapsulation of luteinizing hormone-releasing hormone agonist in poly (lactic-co-glycolic acid) microspheres by spray-drying" (Journal of Controlled Release, Elsevier, Amsterdam, NL, vol. 321, 11 January 2020 (2020-01-11), pages 756-772, XP086135609, ISSN: 0168-3659, DOI: 10.1016/J.JCONREL.2020.01.023) refers to a spray drying technique being developed to prepare injectable and biodegradable poly(lactic-co-glycolic acid) (PLGA) microspheres encapsulating a model luteinizing hormone-releasing hormone agonist (LHRHa)-based peptide, leuprolide.

### Disclosure

### Technical Problems

Therefore, as a result of continuous research to meet the needs of the related art, the present inventors confirmed that microneedles manufactured using a surface-modified microsphere containing a hormone called leuprolide have surprisingly improved residence time in the body and superior sustained-release effect, so they are suitable for use in the treatment of diseases requiring hormone administration, and completed the present invention.

Accordingly, the object of the present invention is to provide microneedles comprising surface-modified microspheres containing a hormone.

Another object of the present invention is to provide a method of preparing microneedles comprising surface-modified microspheres containing a hormone.

Another object of the present invention is to provide a transdermal microneedle patch, which includes the microneedles.

### Technical Solutions

To achieve the objects of the present invention, the present invention provides microneedles comprising surface-modified microspheres containing a hormone.

The "surface-modified" microsphere used herein refers to a microsphere with a surface in which grooves such as a dimple structure which can be seen in a golf ball, wrinkles, etc., are formed. Particularly, in embodiments according to the claimed invention, the "surface-modified" microsphere refers to a microsphere having a surface on which wrinkles are formed.

The "microsphere" used herein is a biodegradable microsphere that serve as a carrier for delivering drugs or other substances into the body. The biodegradable microsphere has a predetermined period of degradation and disappearance in the body based on the degradation mechanism and rate of its main component, a biodegradable polymer. The release of the encapsulated drug within the microsphere occurs over a certain period of time depending on the biodegradation rate of the polymer. The average size of the microsphere is not specifically limited in embodiments outside of the subject-matter of the claims, but it is desirable for it to be of a size suitable for encapsulation into microneedles, approximately 50 µm or less. In the claimed invention, the average size of the microsphere is 10 µm or less. According to the present invention, the terms "microsphere" or "microspheres" are used to refer to "microparticle" or "microparticles", respectively.

In the present invention, the surface-modified microsphere may be formed in a single emulsion type, such as an oil-in-water (O/W) type, a water-in-oil (W/O) type, an oil-in-oil (O/O) type, a solid-in-oil (S/O) type, or a solid-in-water (S/W) type, and preferably, as an oil-in-water (O/W) type emulsion.

In the present disclosure, as a method of preparing microspheres, it may be used a solvent evaporation method, which comprises evaporating an organic solvent used in the preparation of microspheres and performing curing. In addition, a spray drying method and a sonication method may be used. Particularly, in the presently claimed invention, a spray method is used.

When preparing biodegradable microspheres using the spray drying method, a single emulsion, in the case of oil-in water (O/W) microspheres, which is a single emulsion, a non-polar organic solvent that is immiscible with water may be used as an internal oil phase. A biodegradable polymer and a drug can be simultaneously dissolved in the non-polar organic solvent. Specifically, in the presently claimed invention, the microspheres are prepared by a method, which includes i) preparing an oil phase by dissolving a hormone and a surfactant-acting biodegradable polymer in an organic solvent and ii) spray drying the oil phase to obtain surface-modified microspheres. If necessary, the microspheres may be used after washing with a solvent such as ethanol.

In embodiments outside of the claimed invention, the hormone as used herein may be any hormone that is used for diseases requiring continuous hormone administration (requiring a sustained-release effect), and preferably a hormone insoluble in water. Specifically, the hormone may be selected from sex hormones, growth hormones, parathyroid hormone, human chorionic gonadotropin, luteinizing hormone, thyroid-stimulating hormone, follicle-stimulating hormone, gonadotropins, pituitary hormones, adrenocorticotropic hormone, insulin, salmon calcitonin, glucagon, estrogen, parathyroid, desogestrel, ethinyl estradiol, testosterone, oxytocin, prolactin, endorphin, melanocyte-stimulating hormone, thyroxine, triiodinthyronine, somatostatin, adrenaline, glucocorticoid, androgen, aldosterone, progesterone, melatonin, angiotensinogen, gastrin, ghrelin, secretin, cholecystokinin, renin, adenosine, hCG, hPL, leptin, analogues thereof, and mixtures thereof, but the present disclosure is not limited thereto. In embodiments according to the claimed invention, as the hormone, leuprolide was used.

Leuprolide is a type of luteinizing hormone-releasing hormone (LHRH) agonist. LHRH is known as gonadotropin-releasing hormone (GnRH), and is a hypothalamic decapeptide that regulates the reproductive system of vertebrates. By the action of GnRH, the biosynthesis and release of gonadotropins, such as follicle-stimulating hormone (FSH) and luteinizing hormone (LH), are induced. LHRH agonists and antagonists have been shown to be effective in treatment of endometriosis, fibroma, polycystic ovary syndrome, breast cancer, ovarian cancer, and endometrial cancer, treatment of gonadotropin-pituitary desensitization during medical-assisted birth protocols, treatment of benign prostate polymorphism and prostate cancer in men, and treatment of male or female precocious puberty. Currently-used LHRH agonists are peptide compounds that have to generally be administered intravenously or subcutaneously due to their low oral bioavailability. In addition, as LHRH agonists are drugs for chronic diseases, they have to be administered for a long time. Leuprolide has a short half-life when conventionally administered subcutaneously or intramuscularly, resulting in a rapid decrease in the in the bloodstream after administration and thus disappears within a few hours. As a result, there was an inconvenience of having to administer it daily to maintain its efficacy, and this inconvenience was further aggravated by the fact that it was an injectable medication.

The "leuprolide" used herein has the structure of Formula 1 below, and is used in treatment of prostate cancer, breast cancer, endometriosis, uterine leiomyomas, or precocious puberty:

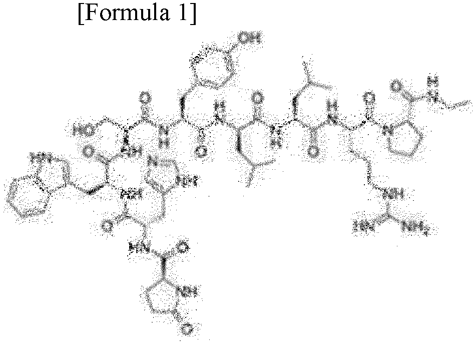

In the present invention, to deliver surface-modified microspheres encapsulating a hormone into the skin, a material for the microneedles may be soluble so that it can be disintegrated by moisture in the skin and may be biocompatible to be absorbed or decomposed without side effects in the body. And the material preferably has sufficient strength to perforate the skin after being manufactured as microneedles.

The microneedles of the present disclosure are water-soluble, that is, soluble in body fluids in the skin. Particularly, the microneedles as presently claimed are soluble in the skin.

In the present invention, the surfactant-acting biodegradable polymer may be naturally biodegraded in the body and thus excreted outside the body. In addition, the surfactant-acting biodegradable polymer may have the function of a surfactant, which can emulsify a hormone. The surfactant-acting biodegradable polymer can be derived from natural sources or synthetically prepared. The surfactant-acting biodegradable polymer of the present disclosure comprises Tweens, poloxamers, poly(lactate-co-glycolate) (PLGA), poly(D,L-lactic acid) (PDLA), or a copolymer of poly(D,L-lactic acid)-polycaprolactone. In the presently claimed invention, PLGA is used. The surfactant-acting biodegradable polymer may have a weight average molecular weight of 5,000 to 1,000,000.

The hormone and the surfactant-acting biodegradable polymer according to the presently claimed invention are comprised in a weight ratio of 1 : 4 to 1 : 8, and preferably 1 : 4. In the above range, a surface with desired grooves (wrinkles) may be effectively formed on the microspheres. Furthermore, primary particles are completely separated each other, and the undesirable aggregation of primary particles may be prevented.

The organic solvent may be, but is not particularly limited to, dichloromethane, methanol, ethanol, chloroform, hexane, ethyl acetate, and a mixture thereof, and preferably, dichloromethane. The ratio of the mixture of the hormone and the surfactant-acting biodegradable polymer and the organic solvent according to the presently claimed invention, that is, the solid content ratio in the spray-drying liquid (oil phase) is 0.6 to 1.3% (w/w), and preferably 1.3% (w/w). In the above range, it is possible to effectively form a surface with desired grooves (wrinkles) on the microspheres. In addition, the separation between primary particles may be clearly shown, and the undesirable aggregation of primary particles may be prevented.

In the above step i), the dissolving time is not particularly limited, and the dissolving of a hormone and a surfactant-acting biodegradable polymer in a solvent may be performed through stirring if necessary.

In the above step ii), the spray drying may be performed using a conventional, commercially-available spray dryer, and as the commercially-available spray dryer, EYELA SD-1000 (Japan), Buchi B-290, (Switzerland), YC-500 (China), Nano Spray Dryer B-90 (Switzerland) or the like is known.

In the spray drying step, the operating conditions of the spray dryer may be determined according to a predetermined value according to the type of spray dryer, but can be changed as necessary.

The spray dryer may spray dry an oil phase (spray drying liquid) by adjusting an inlet temperature, an outlet temperature, and a feed rate. The inlet temperature according to the presently claimed invention is 60 to 80 °C, and preferably 60 °C. Within the above range, depending on the type of spray dryer, the type and properties of the components in a solution to be spray-dried, etc., the inlet temperature can be adjusted as needed. The outlet temperature may be 45 to 95 °C. The outlet temperature can also be appropriately adjusted according to the shape and dried state of particles to be produced.

The feeding into a spray dryer may be performed at a predetermined rate, which may be 1 to 12 mL/min. The feed rate may also be appropriately adjusted according to the type of dryer. The diameter of the nozzle for feeding varies depending on the type of spray dryer, but may range from 0.1 to 0.7 mm.

The pressure of a granulating fluid (e.g., air) in spray drying may be 100 to 150 kPa, and the rate of the fluid flow may be 0.1 to 0.5 m³/min, but the present invention is not limited thereto.

In the present invention, to deliver surface-modified microspheres encapsulating a hormone into the skin, a material for the microneedles may be soluble so that it can be disintegrated by moisture in the skin and may be biocompatible to be absorbed or decomposed without side effects in the body. And the material preferably has sufficient strength to perforate the skin after being manufactured as microneedles.

The microneedles of the present disclosure are water-soluble, that is, soluble in body fluids in the skin. Particularly, the microneedles according to the presently claimed invention are soluble in the skin.

As a soluble material for forming microneedles, one or more biocompatible materials selected from the group consisting of alginic acid, chitosan, collagen, gelatin, hyaluronic acid, chondroitin (sulphate), dextran (sulphate), fibrin, agarose, pullulan, cellulose, polyvinylpyrrolidone (PVP), polyethylene glycol (PEG), polyvinyl alcohol (PVA), a vinylpyrrolidone-vinylacetate copolymer, hydroxypropyl cellulose, hydroxyethylcellulose, hydroxypropylethylcellulose, sodium carboxymethylcellulose, a polyalcohol, cyclodextrin, dextrin, trehalose, glucose, fructose, starch, sucrose, maltose, lactose, lactulose, turanose, melitose, melezitose, dextran, sorbitol, mannitol, and xylitol; derivatives thereof; and mixtures thereof may be used. Particularly, as a soluble material for forming microneedles according to the presently claimed invention, one or more biocompatible materials selected from the group consisting of alginic acid, hyaluronic acid, dextran, cellulose, polyvinylpyrrolidone (PVP), polyethylene glycol (PEG), polyvinyl alcohol (PVA), sodium carboxymethylcellulose, polyalcohol, cyclodextrin, dextrin, trehalose, sucrose, lactose, mannitol, and xylitol; and mixtures thereof are used For instance, a mixture of alginic acid and trehalose is used as the soluble material.

The microneedles of the present invention may further include a plasticizer, a surfactant, and a preservative.

As a plasticizer, for example, polyols such as ethylene glycol, propylene glycol, dipropylene glycol, butylene glycol, glycerin, etc. may be used alone or in combination, but the present invention is not limited thereto. As a surfactant, for example, PEG-8 glyceryl isostearate, PEG-10 glyceryl isostearate, PEG-15 glyceryl isostearate, PEG-20 hydrogenated castor oil, PEG-30 hydrogenated castor oil, PEG-40 hydrogenated castor oil, PEG-60 hydrogenated castor oil, PEG-80 hydrogenated castor oil, and ceteareth-12 may be used alone or in combination, but the present invention is not limited thereto. As a preservative, for example, methyl parahydroxybenzoate, ethyl parahydroxybenzoate, paraoxybenzoic acid, (iso)propylparaoxybenzoic acid, (iso)butylchlorobutanol (chlorobutol), benzalkonium chloride, benzethonium chloride, phenol (p-form), cresol, chlorocresol, dehydroacetic acid, sodium dehydroacetate, sorbic acid, potassium sorbate, sodium sorbate, benzoic acid, and sodium benzoate may be used alone or in combination, but the present invention is not limited thereto.

The needle part of the microneedles according to the present invention may be a conical, pyramidal, spear-shaped, truncated, wedge-shaped, or blade-shaped, or the like, all of which have commonly penetrate the skin. In one embodiment of the present disclosure, microneedles with a pyramid-like needle part, which is structurally stable, are selected.

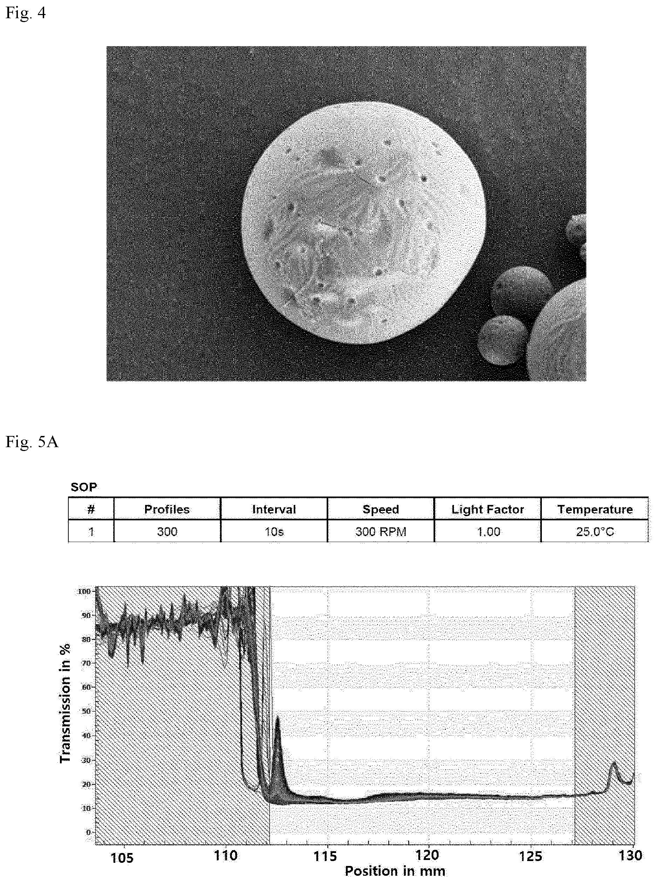

The structures of microneedles 10 and a microneedle patch 100 according to the present invention are shown in FIGS. 4A and 4B. The microneedles 10 of the present invention may comprise a needle part 11 and a matrix layer 12. The needle part 11 has a shape that is easy to penetrate the skin as defined above. The length of the needle part 11 may be 500 to 1,000 µm, and preferably, 750 µm. The thickness of the matrix layer 12 may be 0.1 to 1 mm, and preferably, 0.1 to 0.3 mm. The needle part and the matrix layer encapsulate (contain) a surface-modified microsphere encapsulating a hormone. The microneedle patch 100 of the present disclosure is formed by laminating an adhesive layer 20 on one side of the matrix layer 12, allowing the microneedle patch to be adhered to the skin. In the microneedle patch 100, the adhesive layer part excluding the portion in contact with the microneedles 10 accounts for 50% or less of the entire area of the adhesive layer. The microneedle patch 100 may also comprise a protective film 30 on the adhesive layer.

According to another object of the present disclosure, a method of preparing the microneedles is provided.

The preparing method comprises
a) forming a first solution by dissolving a soluble material in water,
b) preparing surface-modified microspheres containing (encapsulating) a drug,
c) preparing a mixed solution by mixing and homogenizing the first solution and the surface-modified microspheres,
d) filling an engraved microneedle mold with the mixed solution, and
e) drying the mixed solution filled in the mold and separating the resulting product from the mold.

In the manufacturing method of the present invention, the soluble material, the drug, the surface-modified microspheres are defined as described above.

In step a), the first solution may further comprise a plasticizer, a surfactant, and a preservative. The plasticizer, the surfactant, and the preservative, etc., are defined as described above.

A method of preparing the surface-modified microsphere in step b) is defined as described above.

In step c), with respect to 100 parts by weight of the first solution, 0.1 to 20 parts by weight of the surface-modified microsphere is mixed.

In the step of preparing the mixed solution, it is important to ensure that the surface-modified microparticles containing a drug are uniformly distributed within the microneedles. Accordingly, after mixing the first solution and the surface-modified microspheres, vigorous homogenization such as vortexing are applied to achieve a stable and homogeneous dispersion of the microparticles in the mixed solution.

Conditions such as a temperature used in manufacturing the microneedles are not particularly limited as long as the soluble material or surface-modified microspheres can be sufficiently dissolved or mixed without decomposition or deformation.

In step d), the filling of a microneedle mold with the mixed solution may use a method of allowing the mixed solution to stand after applying it, a method of injecting the mixed solution using a centrifuge, a method of injecting the mixed solution by vacuuming to eliminate internal air, or a method of injecting the mixed solution under pressure.

In step e), drying may be performed at room temperature, or may be performed at room temperature to 80 °C using hot air dryer, but the present disclosure is not limited thereto.

According to still another object of the present invention, the presently claimed invention provides a transdermal microneedle patch, which comprises the above-described microneedles including surface-modified microspheres containing a hormone, prepared by the above-described preparing method.

When the hormone is leuprolide, the transdermal microneedle patch may be used for treating and alleviating of prostate cancer, breast cancer, endometriosis, uterine leiomyomas, or precocious puberty.

### Advantageous Effects

Microneedles including surface-modified microspheres containing a hormone according to the present invention exhibit improved residence time in the body and superior sustained-release effect, and are therefore useful for a transdermal delivery system for delivering a hormone requiring continuous administration.

### Description of Drawings

FIG. 1 shows electron micrographs of microspheres prepared in Preparation Example 1.
FIG. 2 is an enlarged electron micrograph of a surface-modified microsphere of Formulation LSD5 prepared in Preparation Example 1.
FIG. 3 is an electron micrograph of conventional microspheres with a smooth surface prepared in Comparative Preparation Example 1.
FIG. 4 is an enlarged electron micrograph of a microsphere with a smooth surface of Formulation L6 prepared in Comparative Preparation Example 1.
FIG. 5A shows the results of measuring, using LUMiSizer, the dispersion stability of leuprolide-containing surface-modified microspheres according to the present invention.
FIG. 5B shows the results of measuring, using LUMiSizer, the dispersion stability of conventional microspheres containing leuprolide with a smooth surface.
FIGS. 6A and 6B are schematic diagrams of microneedles and a microneedle patch according to the present invention.
FIG. 7 shows electron micrographs of microneedles according to the present invention.

### Examples of the Invention

Hereinafter, the configuration and effects of the present invention will be described in further detail with reference to exemplary examples to better explain the present invention. However, the following examples are merely exemplified to more clearly explain the present invention, but the scope of the present invention is not limited by the following examples.

### Preparation Example 1: Preparation of surface-modified microspheres containing leuprolide

Leuprolide-containing microspheres were prepared using a spray drying method. As the leuprolide, leuprolide acetate was used, and as a surfactant-acting biodegradable polymer, PLGA was used. Preparation conditions are shown in Tables 1 and 2 below.

Specifically, leuprolide acetate (ANYGEN Co., Ltd.) and PLGA 503H (Evonik Ltd., Germany) were dissolved in a mixed solvent of dichloromethane and methanol at the amounts shown in Table 1 by stirring for 30 minutes to prepare an oil phase (spray-drying solution). Then dry powder microspheres were obtained from the spray-drying solution using a spray dryer (EYELA SD-1000, Japan) under the same conditions shown in Tables 1 and 2, including a feed rate of 6.5 mL/min, an atomizing air pressure of 130 kPa, and an air flow rate of 0.3 m³/min. Ethanol (99.5%) was added to the microspheres, followed by washing with vortexing for 5 minutes.

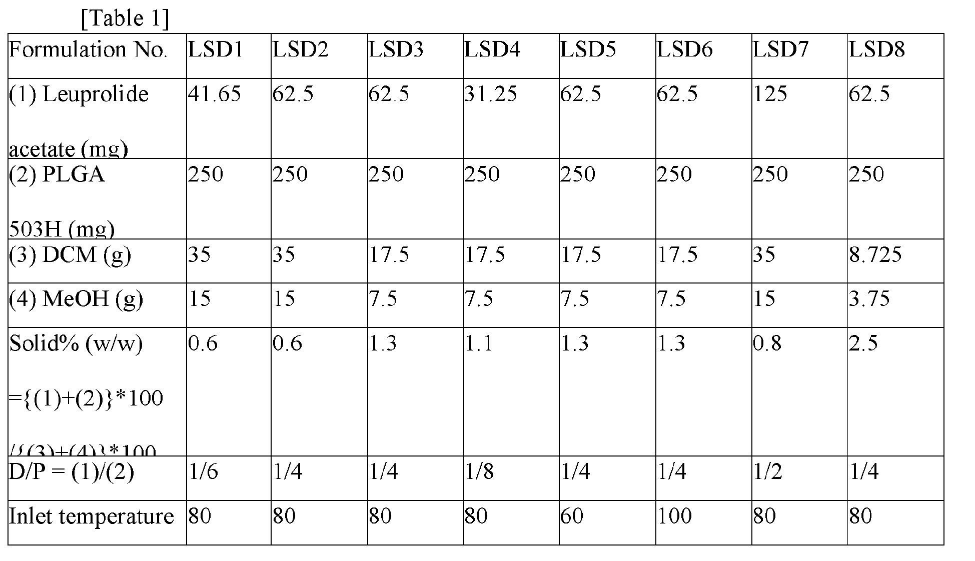

**[Table 2]**

| | |
|---|---|
| Spray dryer | EYELA SD-1000, Japan |
| Inlet Temperature | 60~80 °C |
| Outlet Temperature | 45~95 °C |
| Feed rate | 6.5 mL/min |
| Atomizing air pressure | 130 kPa |
| Air flow rate | 0.3 m³/min |
| Nozzle size | 0.4 mm |

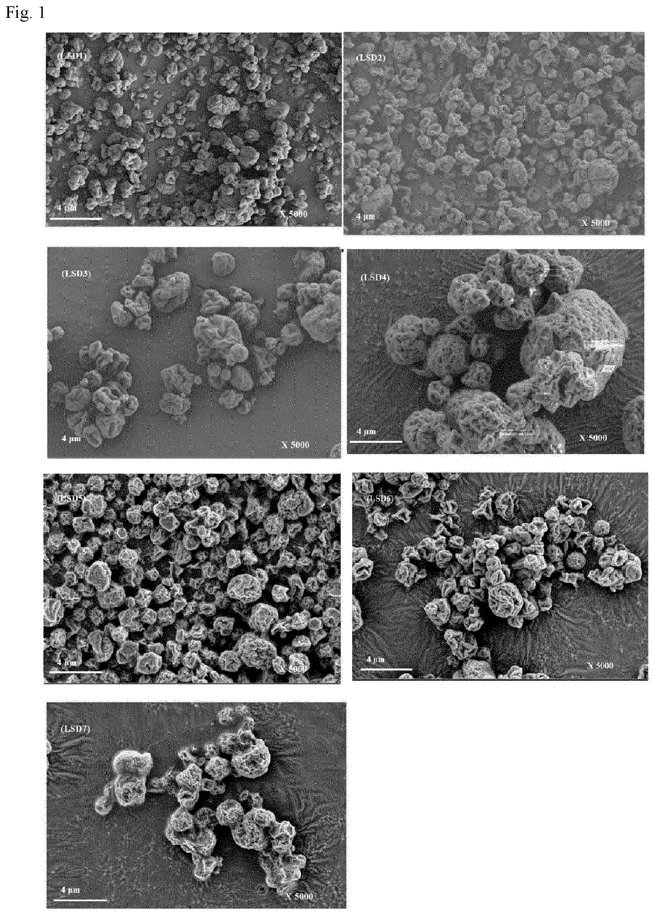

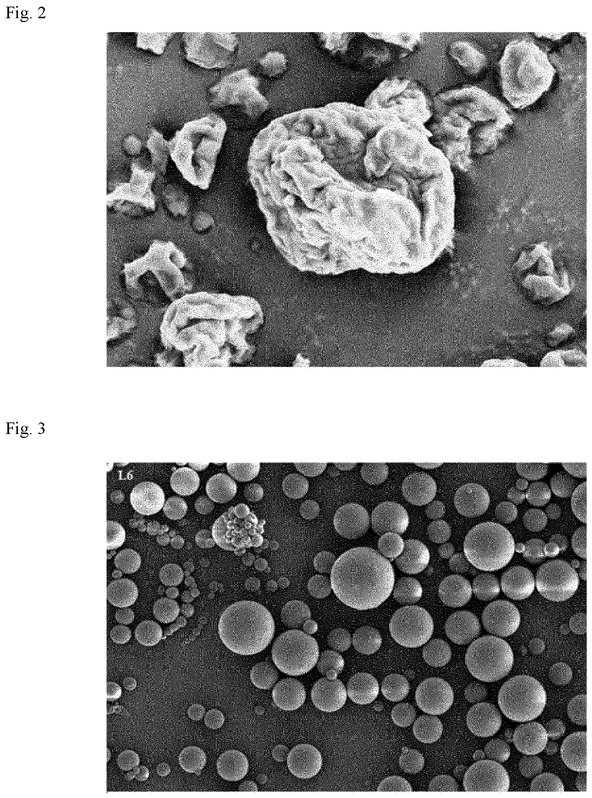

The particle size distribution of prepared leuprolide acetate-containing microspheres of each formulation was measured. The results are shown in Table 3, and the electron micrographs of the microspheres are shown in FIGS. 1 and 2 (excluding Formulation LSD8, in which agglomeration was so severe that it was not possible to take an electron micrograph).

**[Table 3]**

| Formulation | | LSD1 | LSD2 | LSD3 | LSD4 | LSD5 | LSD6 | LSD7 | LSD8 |
|---|---|---|---|---|---|---|---|---|---|
| µm | Dv10 | 0.336 | 0.425 | 0.331 | 0.330 | 0.395 | 0.316 | ND | ND |
| | Dv50 | 7.67 | 7.06 | 5.13 | 6.69 | 7.02 | 6.60 | ND | ND |
| | Dv90 | 34.1 | 42.1 | 23.2 | 19.9 | 22.6 | 625 | ND | ND |
| | Span | 4.399 | 5.910 | 4.447 | 2.923 | 3.166 | 94.65 | ND | ND |
| Yield (%) | | 55.2 | 60.2 | 56.6 | 48.4 | 68.2 | 57.9 | ND | ND |

| | | | | | | | | | |
|---|---|---|---|---|---|---|---|---|---|
| ND : Out of measurement range of instrument because of excessive agglomeration of microsphere particles | | | | | | | | | |

Referring to FIG. 1 and Table 3, in Formulations LSD1 to LSD5, leuprolide acetate-containing microspheres whose surface was modified into a wrinkle structure (grooves) without aggregation between particles were identified, but in Formulations LSD6~LSD8, it was observed that agglomeration of microspheres occurred.

### <Optimization of conditions for preparing surface-modified microspheres>

Comparing Formulations LSD2 and LSD3 with Formulation LSD8, it is observed that an increase in the amount of solvent leads to the formation of microspheres without aggregation. Accordingly, in order to prepare microspheres whose surface is modified into a wrinkle structure (grooves) without agglomeration between particles, the solid content in the spray-drying liquid (oil phase) is 0.6 to 1.3% (w/w), and preferably 1.3% (w/w).

Comparing Formulations LSD4 and LSD3 with Formulation LSD7, when the weight ratio of the drug (hormone) to the polymer (PLGA) decreases, microspheres without agglomeration are formed. Accordingly, in order to prepare microspheres whose surface is modified into a wrinkle structure (grooves) without agglomeration between particles, the appropriate weight ratio of the drug (hormone) to the polymer (PLGA) is 1 : 4 to 1 : 8, and preferably 1 : 4.

Comparing Formulations LSD5 and LSD3 with Formulation LSD6, when the inlet temperature of the spray dryer decreases, microspheres without agglomeration are formed. Accordingly, in order to prepare microspheres whose surface is modified into a wrinkle structure (grooves) without agglomeration between particles, the inlet temperature is 60 to 80 °C, and preferably 60 °C.

### Comparative Preparation Example 1: Preparation of leuprolide-containing microspheres with smooth surface

Leuprolide-containing microspheres with a smooth surface were prepared through a solvent evaporation method using an oil-in-water (O/W). Conditions for a formulation composition, an oil phase, a water phase, a solvent composition, a homogenizer, and a mechanical stirrer are shown in Table 4 below.

Specifically, 100 mg of leuprolide acetate and 600 mg of PLGA 503H (Evonik Ltd., Germany) were dissolved in a mixed solvent including 11.2 g of dichloromethane and 2.9 g of methanol to prepare an oil phase. Then the oil phase was mixed with 400 mL of a water phase of a 0.5% polyvinyl alcohol (PVA 500, OCI Company, Ltd., Korea) solution using a homogenizer at 10,000 rpm for 2 minutes, thereby forming an O/W emulsion. The O/W emulsion was stirred using a mechanical stirrer at 1,000 rpm for 3 hours to evaporate the organic solvent, thereby forming microspheres. To remove remaining PVA and drug particles which are not encapsulated by a polymer, centrifugation was performed at x 200 g for 5 minutes, and lyophilization was performed, thereby obtaining powdery microspheres. The images of the microspheres are shown in FIGS. 3 and 4.

**[Table 4]**

| Formulation | | L6 |
|---|---|---|
| Drug | Leuprolide acetate (mg) | 100 |
| Polymer | PLGA 503H (mg) | 600 |
| Oil phase solvent | DCM (g) | 11.2 |
| | MeOH (g) | 2.9 |
| Water phase | PVA aqueous solution (w/v, %) | 0.5 %, 400 mL |

### Experimental Example 1: Analysis of dispersion stability of surface-modified microspheres

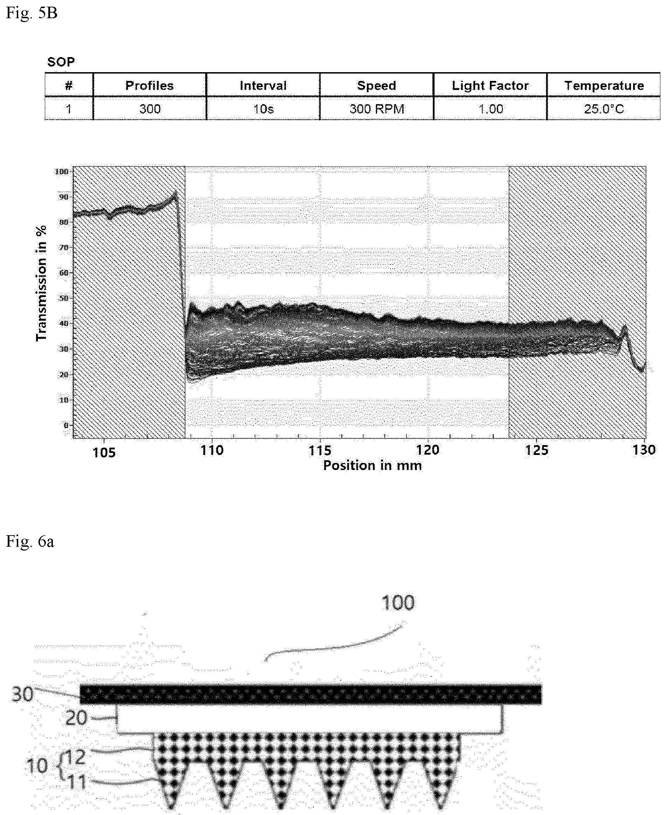

The dispersion stability of the surface-modified (wrinkle-structured) microspheres (LSD5) prepared in Preparation Example 1 and the microspheres with a smooth surface (Formulation L6) was measured using LUMiSizer, respectively. The results are shown in FIGS. 5A and 5B and Table 5:

**[Table 5]**

| Formulation | Instability test | Mean RCA in g | Time in s |
|---|---|---|---|
| L6 | 0.284 | 11.70 | 3,000 |
| LSD5 | 0.032 | 12.04 | 2,989 |

It can be confirmed that the surface-modified microspheres according to the present invention have a low instability index value, indicating high dispersion stability.

### Preparation Example 2: Preparation of microneedles comprising surface-modified microspheres

Microneedles of Example 1 comprising surface-modified (wrinkle-structured) microspheres containing leuprolide acetate (Formulation LSD5) prepared in Preparation Example 1 were prepared. Also microneedles of Comparative Example 1 comprising microspheres with a smooth surface (Formulation L6) prepared in Comparative Preparation Example 1 were prepared.

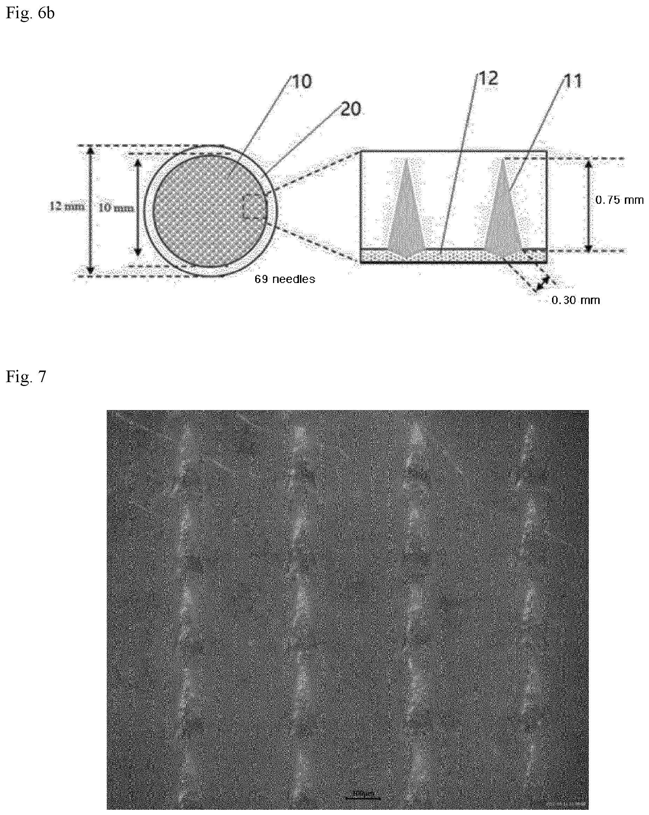

Specifically, after a first solution was prepared with 1.0 g of sodium alginate (SUNFINE GLOBAL), 1.0 g of trehalose (SUNFINE GLOBAL), and 33 g of H₂O. Then 9.9 g of the first solution and 0.1 g of the surface-modified microspheres of Formulation LSD5 prepared in Preparation Example 1, or the microspheres with a smooth surface of Formulation L6 prepared in Comparative Preparation Example 1 were mixed by vortexing for 5 minutes or more to disperse for homogenization. The resulting mixture was then filled into a pyramid-like engraved silicone mold with a depth of 750 µm. The mold was placed in a desiccator, subjected to a reduced pressure of -0.04 Mpa for 30 minutes, and dried in a hot air dryer at 35 °C for 2 hours and 10 minutes. The dried microneedles were collected using adhesive tape, and the edges were rounded by cutting with scissors to match the patch shape (FIG. 6B). The electron micrographs of the completed microneedles were taken and shown in FIG. 7.

As shown in FIG. 7, it can be confirmed that the microneedles are well formed.

In addition, the strength of the completed microneedles of Example 1 was evaluated under the conditions of Table 6 using a texture analyzer, and the results are shown in Table 7.

**[Table 6]**

| Measurement conditions (TA.XT Plus Texture analyzer) | |
|---|---|
| Mode | Compression |
| Pre-Test Speed | 1.00mm/sec |
| Test Speed | 0.1mm/sec |
| Post-Test Speed | 10.00 mm/sec |
| Distance | 0.800 mm |
| Trigger Force | 0.049 N |

**[Table 7]**

| | Strength per needle | | | |
|---|---|---|---|---|
| | No. | Strength (N) | Average | STD |
| Comparative | 1 | 3.50 | 3.54 | 0.07 |
| Example 1 | 2 | 3.62 | | |
| | 3 | 3.51 | | |
| Example 1 | 1 | 3.37 | 3.45 | 0.07 |
| | 2 | 3.49 | | |
| | 3 | 3.50 | | |

As shown in Table 6, it can be confirmed that the strength of the microneedles of Example 1 is 3.45 on average, which is a strength sufficient for skin penetration.

### Experimental Example 2: Analysis of residence time in the subcutaneous body of drug (hormone)

The hairless dorsal skin tissue of a 8-week-old male SD rat was collected and mounted on an *in-vitro* Franz cell permeation tester ((Phoenix DB-6, Teledyne, US) 400 rpm, 37 °C). Each of the microneedle patches of Example 1 and Comparative Example 1, prepared in Preparation Example 2, was attached to the dorsal skin for 0.5 minutes and then removed. Samples from the dorsal skin tissue was taken at 0, 1, 12, or 24 hours. The surface of the sampled skins was wiped with an alcohol swab and extracted by shaking with an HPLC mobile phase. The supernatant from the resulting extracts was quantitatively analyzed using HPLC. The results are presented in Table 8.

**[Table 8]**

| Units: % | Example 1 | Comparative Example 1 |
|---|---|---|
| Initial | 100.0 | 100.0 |
| 1hr | 30.9 | 16.3 |
| 24hr | 25.2 | 3.3 |

As shown in Table 8, after 24 hours had elapsed, when the microneedle patch comprising the surface-modified microspheres of Example 1 was used, the subcutaneous residual amount of leuprolide was high at 25.2% relative to the initial amount. However, when the microneedle patch including microspheres with a smooth surface of Comparative Example 1 was used, the subcutaneous residual amount of leuprolide was very low at 3.3% relative to the initial amount.

Injected microspheres into skin may be removed from the subcutaneous tissue due to skin elasticity. It can be observed that the surface-modified microspheres in Example 1, due to their surface roughness, are not detached from the subcutaneous tissue and exhibit higher residence time in the body compared to the microneedles with a smooth surface in Comparative Example 1. Accordingly, it can be seen that the microneedles comprising surface-modified microspheres according to the present invention have increased residence time in the body, ultimately achieving long-acting effect (sustained-release effect) of a hormone within the microspheres.

### Description of reference numerals

10 : Microneedles
11: Needle part
12: Matrix layer
20: Adhesive layer
30: Protective film
100: Microneedle patch

## Claims

1. Microneedles being soluble in a skin and having improved residence time in a body, the microneedles comprising surface-modified microparticles encapsulating leuprolide,
wherein the surface-modified microparticles are biodegradable and have a surface on which wrinkles are formed,
wherein the surface-modified microparticles are prepared by a spray drying method comprising:
i) preparing an oil phase by dissolving leuprolide and a surfactant-acting biodegradable polymer in an organic solvent; and
ii) spray drying the oil phase to obtain the surface-modified microparticles,
wherein in the oil phase, the solid content of leuprolide and the surfactant-acting biodegradable polymer ratio is 0.6 to 1.3% (w/w), the weight ratio of leuprolide to the surfactant-acting biodegradable polymer is 1 : 4 to 1 : 8, and an inlet temperature of spray drying is 60 to 80 °C,
wherein the average size (D50) of the surface-modified microparticles is 10*µ*m or less,
wherein the surfactant-acting biodegradable polymer is poly(lactate-co-glycolate) (PLGA), and
wherein a soluble material for forming the microneedles is any one selected from the group consisting of alginic acid, hyaluronic acid, dextran, cellulose, polyvinylpyrrolidone (PVP), polyethylene glycol (PEG), polyvinyl alcohol (PVA), sodium carboxymethylcellulose, polyalcohol, cyclodextrin, dextrin, trehalose, sucrose, lactose, mannitol, xylitol and mixtures thereof.

2. The microneedles of claim 1, wherein the soluble material that forms the microneedles is a mixture of alginic acid and trehalose.

3. A transdermal microneedle patch comprising the microneedles comprising the surface-modified microparticles encapsulating leuprolide according to claim 1.

## Patentansprüche

1. Mikronadeln, die in Haut auflösbar sind und eine verbesserte Verweilzeit in einem Körper aufweisen, wobei die Mikronadeln oberflächenmodifizierte Mikropartikel, die Leuprolid einkapseln, umfassen,
wobei die oberflächenmodifizierten Mikropartikel biologisch abbaubar sind und eine Oberfläche aufweisen, auf der Falten gebildet sind,
wobei die oberflächenmodifizierten Mikropartikel durch ein Sprühtrocknungsverfahren hergestellt werden, das Folgendes umfasst:
i) Herstellen einer Ölphase durch Lösen von Leuprolid und eines oberflächenaktiv wirkenden, biologisch abbaubaren Polymers in einem organischen Lösungsmittel; und
ii) Sprühtrocknen der Ölphase, um die oberflächenmodifizierten Mikropartikel zu erhalten,
wobei in der Ölphase das Verhältnis des Feststoffgehalts von Leuprolid und des oberflächenaktiv wirkenden, biologisch abbaubaren Polymers im Bereich von 0,6 bis 1,3 % (w/w) liegt, das Gewichtsverhältnis von Leuprolid zum oberflächenaktiv wirkenden, biologisch abbaubaren Polymer im Bereich von 1 : 4 bis 1 : 8 liegt und eine Einlasstemperatur des Sprühtrocknens im Bereich von 60 bis 80 °C liegt,
wobei die durchschnittliche Größe (D50) der oberflächenmodifizierten Mikropartikel 10 µm oder kleiner ist,
wobei der oberflächenaktiv wirkende, biologisch abbaubare Polymer Poly(lactid-coglycolid) (PLGA) ist, und
wobei ein auflösbares Material zum Bilden der Mikronadeln irgendeines ist, das aus der Gruppe ausgewählt wird, die aus Alginsäure, Hyaluronsäure, Dextran, Cellulose, Polyvinylpyrrolidon (PVP), Polyethylenglykol (PEG), Polyvinyl-Alkohol (PVA), Natriumcarboxymethylcellulose, Polyalkohol, Cyclodextrin, Dextrin, Trehalose, Saccharose, Lactose, Mannitol, Xylitol und Gemischen davon besteht.

2. Mikronadeln nach Anspruch 1, wobei das auflösbare Material, das die Mikronadeln bildet, ein Gemisch aus Alginsäure und Trehalose ist.

3. Hautdurchdringendes Mikronadelpflaster, das die Mikronadeln, die die oberflächenmodifizierten Mikropartikel, die Leuprolid einkapseln, enthalten, nach Anspruch 1 enthält.

## Revendications

1. Micro-aiguilles étant solubles dans une peau et ayant un temps de séjour amélioré dans un corps, les micro-aiguilles comportant des microparticules modifiées en surface encapsulant un leuprolide,
dans lesquelles les microparticules modifiées en surface sont biodégradables et ont une surface sur laquelle des rides sont formées,
dans lesquelles les microparticules modifiées en surface sont préparées par un procédé de séchage par pulvérisation comportant de :
i) préparer une phase huileuse en dissolvant un leuprolide et un polymère biodégradable agissant comme tensioactif dans un solvant organique ; et
ii) sécher par pulvérisation la phase huileuse pour obtenir les microparticules modifiées en surface,
dans lesquelles, dans la phase huileuse, la teneur en matières solides du leuprolide et le rapport de polymère biodégradable agissant comme tensioactif est de 0,6 à 1,3 % (w/w), le rapport pondéral du leuprolide sur le polymère biodégradable agissant comme tensioactif est de 1:4 à 1:8, et une température d'entrée du séchage par pulvérisation est de 60 à 80 °C,
dans lesquelles la taille moyenne (D50) des microparticules modifiées en surface est de 10 *µ*m ou moins,
dans lesquelles le polymère biodégradable agissant comme tensioactif est le poly(lactate-co-glycolate) (PLGA), et
dans lesquelles un matériau soluble pour former les micro-aiguilles est un matériau quelconque choisi parmi le groupe constitué d'acide alginique, d'acide hyaluronique, de dextrane, de cellulose, de polyvinylpyrrolidone (PVP), de polyéthylène glycol (PEG), d'alcool polyvinylique (PVA), de carboxyméthylcellulose sodique, d'un polyalcool, de cyclodextrine, de dextrine, de tréhalose, de saccharose, de lactose, de mannitol, de xylitol et de mélanges de ceux-ci.

2. Micro-aiguilles selon la revendication 1, dans lesquelles le matériau soluble qui forme les micro-aiguilles est un mélange d'acide alginique et de tréhalose.

3. Timbre transdermique à micro-aiguilles comportant les micro-aiguilles comportant les microparticules modifiées en surface encapsulant un leuprolide selon la revendication 1.
